# EUROPÄISCHE PATENTSCHRIFT

(11) **EP 1 299 091 B1**
(45) Veröffentlichungstag und Bekanntmachung des Hinweises auf die Patenterteilung: **11.10.2006**
(21) Anmeldenummer: 01960496.6
(22) Anmeldetag: 12.07.2001
(51) Int. Cl.: A61K 9/70, A61K 38/55

(54) **MATRIXKONTROLLIERTES TRANSDERMALES SYSTEM FÜR STABILE DERIVATE DER ACE-HEMMER**
MATRIX CONTROLLED TRANSDERMAL SYSTEM FOR STABILE DERIVATIVES OF ACE INHIBITORS
SYSTEME TRANSDERMIQUE A REGULATION MATRICIELLE POUR DERIVES STABLES DES INHIBITEURS DE L'ECA

(30) Priorität: 12.07.2000 DE 10033855
(43) Veröffentlichungstag der Anmeldung: 09.04.2003
(73) Patentinhaber: HEXAL AG, 83607 Holzkirchen (DE)
(72) Erfinder: KLOKKERS, Karin, 83607 Holzkirchen (DE); KRAMER, Kai-Thomas, 83607 Holzkirchen (DE); FISCHER, Wilfried, 83607 Holzkirchen (DE); SENDL-LANG, Anna, 83607 Holzkirchen (DE)
(74) Vertreter: Boeters, Hans Dietrich
(86) Internationale Anmeldenummer: PCT/EP2001/008071
(87) Internationale Veröffentlichungsnummer: WO 2002/003970

(56) Entgegenhaltungen:
- EP-A- 0 439 430
- EP-A- 0 452 837
- WO-A-94/01093
- WO-A-96/29999

## Beschreibung

Die Erfindung betrifft ein stabiles, wirkstoffhaltiges transdermales therapeutisches System zur Anwendung von stabilen Derivaten solcher ACE-Hemmern, deren Metaboliten eine Dicarbonsäure darstellen. Stabile Derivate dieser ACE-Hemmer werden durch Salzbildung mit einer Säure oder Veresterung der Dicarbonsäuren erzielt.

Die Langzeittherapie der Hypertonie mit Angiotensin Converting Enzyme-Hemmern (ACE-Hermmer) nimmt einen immer breiteren Raum ein.
ACE-Hemmer sind bei guter Verträglichkeit für ihre zuverlässige Wirksamkeit bekannt. Bisher auf dem Markt erhältlich sind nur orale Darreichungsformen der ACE-Hemmer; wie Tabletten oder Kapseln. Der Nachteil oraler Darreichungsformen besteht darin, daß der Patient jeden Tag mindestens eine Tablette oder Kapsel schlucken muß und der Blut-Plasmaspiegel immer gewissen Schwankungen unterworfen ist. Ein gleichbleibender Plasmaspiegel ist mit oralen Darreichungsformen kaum zu gewährleisten.

Die transdermale Applikation dagegen bietet für ACE-Hemmer eine Reihe von Vorteilen:
die Haut ist unbegrenzt zugänglich,
es erfolgt kein Milieuwechsel wie bei der peroralen Applikation,
die Handhabung ist einfach und bequem,
es genügt normalerweise eine einmalige Gabe statt mehrfacher täglicher Gaben,
die Patienten-Compliance ist wesentlich besser,
es ist eine kontinuierliche Langzeittherapie möglich,
die Freisetzung des Wirkstoffes erfolgt annähernd gemäß einer Kinetik 0-ter Ordnung, eine Therapie kann schneller unterbrochen werden,
es wird ein konstanter Plasmaspiegel über längere Zeit sichergestellt,
ein anfangs zu hoher Plasmaspiegel, wie bei intravenöser Applikation wird vermieden und
aufgrund der Umgehung der 1. Passage bedarf es teilweise einer niedrigeren Dosierung als bei der oralen Gabe, wodurch eine geringere Nebenwirkungsrate auftritt und
die Gefahr von Über- oder Unterdosierung geringer ist.
Aus WO-A1-93/23019 ist bereits ein transdermales Reservoirsystem mit einem Gehalt an einem ACE-Hemmer und
   a) einer undurchlässigen Abdeckschicht (Backing Layer)
   b) einem schichtartigen Element mit Hohlraum,
   c) einem die Wirkstoffabgabe steuernden Mittel (claim 1) und
   d) einer abziehbaren Deckschicht (Release Liner) auf Papierbasis (Seite 12 Zeile 7/8) bekannt.

Transdermale Systeme mit einem Gehalt an einem ACE-Hemmer werden ferner in EP-A-0 439 430 (Reservoir-TTS) und EP-A-0 468 875 (Matrix-TTS) beschrieben, wobei nach EP-A-0 468 875 Silikon-Elastomere als Matrixmaterial verwendet werden. EP-A-452 837 beschreibt eine Matrix für Pflaster, das u.a. ACE-Hemmer als Wirkstoffe enthält. Allerdings werden als mögliche ACE-Hemmer konkret Delaprilhydrochlorid, Enalaprilmaleat, Captopril, Alacepril und (R)-3-[(S)-1-Carboxy-5-(4-piperidyl)-pentyl]-amino-4-oxo-2,3,4,5-tetrahydro-1,5-benzothiazepin-5-Essigsäure genannt. WO 96/29999 beschreibt ein TTS mit einer Matrix auf Basis von Polyisobutylen oder Butylkautschuk mit einem Gehalt an Trandolapril und/ oder Ramipril.

Es hat sich nun gezeigt, daß für ein Matrix-TTS von bestimmten unstabilisierten ACE-Hemmern die Stabilität des Wirkstoffes im Pflaster den Anforderungen nicht genügt.

Die Zersetzung des ACE-Hemmers in der Matrix erfolgt in einem derart großen Ausmaß, daß schon nach kurzer Lagerzeit der Gehalt an den Zersetzungsprodukten so hoch ist, daß die Zulässigkeitsgrenze an Abbauprodukten weit überschritten wird. Auch kann mit unstabilisierten ACE-Hemmern keine ausreichende in-vitro-Permeation durch die Haut erreicht werden.

Die Aufgabe der vorliegenden Erfindung ist die Bereitstellung eines Matrix-TTS mit einem Gehalt an stabilem Derivat von ACE-Hemmern, dessen Stabilität bezüglich des Abbaus des Wirkstoff den gesetzlichen Anforderungen entspricht und dessen in-vitro-Hautpermeation deutlich gesteigert ist. Der Wirkstoffgehalt soll über einen längeren Zeitraum stabil sein und praktisch keinen Zersetzungsvorgängen unterworfen sein. Die Klebkraft des Matrix-Pflasters soll für eine Tragedauer von mind. 3 Tage ausreichend sein.

Überraschenderweise wurde nun gefunden, daß die Salze der aktiven Metaboliten (= Dicarbonsäure) von ACE-Hemmern, die durch Umsetzung der Dicarbonsäure mit starken Säuren (1:1) gebildet werden, einerseits weitgehend stabil gegen Zersetzung sind und andererseits hervorragende Hautpermeation aufweisen. Die Salze der aktiven Metaboliten können dabei in der Matrixschicht in-situ gebildet werden.

Eine Stabilisierung der ACE-Hemmer kann erstaunlicherweise auch durch eine zweifache Veresterung ihrer Metaboliten erreicht werden. Durch die damit verbundene Erhöhung der Lipophilie des ACE-Hemmers läßt sich eine ausgezeichnete Hautpermeation erzielen.

In manchen Fällen sind die stabilen Derivate der ACE-Hemmer derart hygroskopisch, daß sich das Pflaster schon nach kurzer Zeit von der Haut ablöst. Diese Schwierigkeit wurde gelöst, indem über das eigentliche Matrixpflaster, bestehend aus einer für den Wirkstoff undurchlässigen Deckschicht, einer wirkstoffhaltigen, selbstklebenden Matrix und einer abziehbaren Schutzschicht, noch ein "Overtape" aufgebracht wurde. Das "Overtape" kann das eigentliche Matrixpflaster an allen Seiten überragen. Unter "Overtape" versteht man einen Verbund aus einer Deckschicht und einer Kleberschicht.

Die der Erfindung zugrundeliegende Aufgabe wird nun durch ein matrixkontrolliertes transdermales therapeutisches System mit einem Gehalt an mindestens einem stabilen Derivat eines ACE-Hemmers gelöst. Die Klebkraft des Pflasters und dessen Trageeigenschaften können gegebenenfalls durch das Aufbringen eines "Overtapes" entscheidend verbessert werden.

Das erfindungsgemäße transdermale therapeutische System kann aus einer für den Wirkstoff undurchlässigen Deckschicht (5), aus einer oder mehreren den Wirkstoff und/oder fakultative Permeationsförderer enthaltenden, selbstklebenden Matrixschicht(en) (6) oder einer oder mehreren Matrixschicht(en) (9), die mit einem Haftkleber (8) beschichtet sind und einer abziehbaren Schutzschicht (7) bestehen. Im Falle hygroskopischer, stabilisierter ACE-Hemmer wird ein "Overtape" (1), das aus einem Verbund aus einer Deckschicht (3) und einer Haftklebeschicht (4) zusammengesetzt ist, verwendet. Das "Overtape" (1) kann das übrige System (2) an allen Seiten überragen.

In dem erfindungsgemäßen transdermalen therapeutischen System kann mindestens ein stabiles Salz der aktiven Metaboliten (= Dicarbonsäure) eines ACE-Hemmers, das auf einer Reaktion einer sauer reagierenden Verbindung mit der Dicarbonsäure basiert, verwendet werden.

Als sauer reagierende Verbindungen kommen in Frage: anorganische Säuren, wie z. B. Salzsäure, Bromwasserstoffsäure, Iodwasserstoffsäure, Salpetersäure, Schwefelsäure, Phosphorsäure, organische Carbonsäuren, wie z. B. Salicylsäure, Maleinsäure, Adipinsäure, Sorbinsäure, Malonsäure, 1,4-Butandisäure, Äpfelsäure, Pivalinsäure, Bemsteinsäure, Nicotinsäure, Isonicotinsäure, Furan-2-carbonsäure, Dichloressigsäure, Benzoesäure, Fettsäuren, wie z. B. Laurinsäure, Myristylsäure, Ölsäure, aliphatische Sulfönsäuren, wie z.B. Methan-, Ethan-, Propan-, Isopropan-, Butan-, Isobutan-, Pentan-, Isopentan-, Hexan-, Heptan-, Octan-, Nonan-, Decan-, Undecan-, Dodecansulfonsäure oder aromatische Sulfonsäuren, wie z.B. Toluol- oder Benzolsulfonsäure, insbesondere Methan-, Toluol- oder Benzolsulfonsäure. Die bevorzugt verwendete Säure ist die Methansulfonsäure.

Es können die stabilen Salze der aktiven Metabolite (= Dicarbonsäure) der ACE-Hemmer in der Matrix in-situ gebildet werden, indem die entsprechenden sauer reagierenden Verbindungen und die Dicarbonsäuren zusammen in die Matrix eingearbeitet werden.
Die stabilen Salze der Metaboliten der ACE-Hemmer können aber auch direkt in die Matrix eingebracht werden.

In einer alternativen Ausführungsform des erfindungsgemäßen transdermalen therapeutischen Systems kann mindestens ein stabiler zweifacher Ester eines ACE-Hemmer-Metaboliten verwendet werden. Für den Alkylrest des Esters kommen in Frage die Methyl-, Ethyl-, Propyl-, Isopropyl-, Butyl-, tert.-Butyl-, Pentyl-, Hexyl-, Heptyl-, Octyl-, Nonan-, Decangruppe und ihre Isomeren. Bevorzugt wird der Ethylester.

Das erfindungsgemäße transdermale therapeutische System kann als Wirkstoff die stabilisierten Formen solcher ACE-Hemmer enthalten, deren aktive Metaboliten eine Dicarbonsäure darstellen. Beispiele sind Imidapril, Fosinopril, Moexipril, Perindopril, Ramipril, Spirapril, Cilazapril, Benazepril und/ oder Trandolapril. Bevorzugt werden die Monosulfonsäuresalze sowie die Diester von Trandolaprilat und/ oder Ramiprilat als Wirkstoffkomponenten verwendet.

Der Gehalt an ACE-Hemmern kann 2-25 Gew.%, insbesondere 10 -15 Gew.%, bezogen auf das Matrixgewicht, betragen.

Der Gehalt an Säure ist äquimolar zum Metaboliten (= Dicarbonsäure) des ACE-Hemmers und hängt somit vom Molekulargewicht der Dicarbonsäure ab.

Als undurchlässige Deckschicht kommen Folien aus Acetal, Acrylat, Acrylonitril-Butadien-Styrol, Acrylonitril (Methyl Methacrylat) Copolymer, Acrylonitril Copolymer, Ethylen Ethyl Acrylat, Ethylen Methyl Acrylat, Ethylen Vinyl Acetat, Ethylen Vinyl Acetat Copolymer, Ethylen Vinylalkohol Polymer, Ionomere, Nylon (Polyamid), Nylon (Polyamid) Copolymer, Polybutylen, Polycarbonat, Polyester, Polyethylenterephthalat, thermoplastisches Polyester Copolymer, Polyethylen Copolymer (high density), Polyethylen (high-molecular-weight, high-density), Polyethylen (intermediate-molecular-weight, high-density), Polyethylen (linear low density), Polyethylen (low density), Polyethylen (medium density), Polyethylenoxid, Polyimid, Polypropylen, Polypropylen (coated), Polypropylen (oriented), Polystyrol, Polyurethan, Polyvinylacetat, Polyvinylchlorid, Polyvinylidenchlorid und/ oder Styrol- Acrylonitril in Frage, die bei Bedarf metallisiert oder pigmentiert werden können. Als für den Wirkstoff undurchlässige Deckschicht wird Polyurethan bevorzugt.

Als Deckschicht für das Overtape kommen mikroperforierte Folien aus oben genannten Materialien und Membranen aus Polyurethan, Polyethylen, Polypropylen, Polyester, Coextrudate aus Ethylvinylalkohol/Polyethylen oder Polyethylen/Polypropylen in Frage. Als Deckschicht für das Overtape werden Polyurethan Ester und Polyurethan Ether bevorzugt.

Für die Haftklebeschicht insbesondere des Overtapes kann man ein druckempfindliches Klebemittel beispielsweise auf Polyurethanbasis, Polyisobutylenbasis, Polyvinyletherbasis, Polyacrylatbasis oder ein Gemisch aus diesen wählen. Bevorzugt werden Kleber auf Acrylat- und Polyisobutylenbasis verwendet.

Bei den Klebemitteln auf Polyacrylatbasis kann es sich um ein beliebiges Homopolymer, Copolymer oder Terpolymer, bestehend aus verschiedenen Acrylsäurederivaten handeln.

So können die Polyacrylate Polymere eines oder mehrerer Monomere von Acrylsäuren und anderen copolymerisierbaren Monomeren sein. Außerdem können die Polyacrylate Copolymere von Alkylacrylaten und/ oder -methacrylaten und/ oder copolymerisierbaren sekundären Monomeren oder Monomeren mit funktionellen Gruppen umfassen. Verändert man den Betrag jeder Sorte, die als Monomer hinzugefügt ist, können die kohäsiven Eigenschaften der daraus resultierenden Acrylatpolymere verändert werden. Im allgemeinen besteht das Acrylatpolymer aus mindestens 50 Gew.-% eines Acrylat-, Methacrylat-, Alkylacrylat- oder Alkylmethacrylat-Monomers, 0 bis 20 % eines funktionellen Monomers, copolymerisierbar mit Acrylat, und 0 bis 50 % eines anderen Monomeren.

Im folgenden sind verschiedene Acrylatmonomere, wie z.B. Acrylsäure, Methacrylsäure, Butylacrylat, Butylmethacrylat, Hexylacrylat, Hexylmethacrylat, Isooctylacrylat, Isooctylmethacrylat, Glycidylmethacrylat, 2-Hydroxyethylacrylat, Methylacrylat, Methylmethacrylat, 2-Ethylhexylacrylat, 2-Ethylhexylmethacrylat, Decylacrylat, Decylmethacrylat, Dodecylacrylat, Dodecylmethacrylat, Tridecylacrylat und Tridecylmethacrylat aufgeführt, die alleine oder in Mischungen polymerisiert sein können.

Zusätzlich können funktionelle Monomere, die mit den oben genannten Acrylaten, copolymerisierbar sind, wie beispielsweise Acrylsäure, Methacrylsäure, Maleinsäure, Maleinanhydrid, Hydroxyethylacrylat, Vinylacetat, Hydroxypropylacrylat, Acrylamid, Dimethylacrylamid, Acrylnitril, Dimethylaminoethylacrylat, Dimethylaminoethylmethacrylat, tert.-Butylaminoethylacrylat, tert.-Butylaminoethylmethacrylat, Methoxyethylacrylat und Methoxyethylmethacrylat, zur Copolymerisierung eingesetzt werden.

Weitere Einzelheiten und Beispiele für druckempfindliche Acrylate, welche für die Erfindung geeignet sind, sind in Satas Handbook of Pressure Sensitive Adhesive Technology "Acrylic Adhesives", 2^{nd} ed., pp. 396-456 (D. Satas, ed.), Van Nostrand Reinhold, New York (1989) beschrieben.

Der Gehalt an Klebemitteln der selbstklebenden Matrix kann 50 - 90 Gew.%, insbesondere 70 - 80 Gew. %, bezogen auf das Matrixgewicht, betragen:

Für die Matrix werden die medizinisch üblichen Matrixbildner wie Polyacrylat, Polyisobutylen, Kautschuk, kautschukähnliche synthetische Homo-, Co- oder Blockpolymere, Styrol/ Butadien- Copolymerisat oder ein Gemisch aus diesen, wie sie im Stand der Technik vorgesehen werden, verwendet. Bevorzugt wird eine selbstklebende Matrix aus Polyacrylat und/ oder Polyisobutylen verwendet, wobei Matrixbildner und Klebemittel eins sind.

Für die abziehbare Schutzschicht kommen Polyester, Polyethylen, Polypropylen, Polysiloxan, Polyacrylat, Ethylenvinylacetat, Polyurethan, Polyisobuten oder Papier, meistens mit Silikon- und /oder Polyethylen beschichtet, oder ein Gemisch aus diesen in Betracht.

Als Permeationsförderer lassen sich gegebenenfalls gesättigte und/ oder ungesättigte Fettalkohole mit jeweils 8- 18 C- Atomen; Teebaumöl; gesättigte und/ oder ungesättigte cyclische Ketone; Alkyl- Methylsulfoxide; gesättigte und/ oder ungesättigte Fettsäuren mit jeweils 8- 18 C- Atomen; deren Ester und Salze; natürliches Vitamin E; synthetisches Vitamin E und/ oder Vitamin E- Derivate; Sorbitanfettsäureester und ethoxylierte Sorbitanfettsäureester; Azone (Laurocapram); 1-Alkylpyrrolidon; Blockcopolymere von Polyethylenglykol und Dimethylsiloxan mit kationischer Gruppe an einem Ende; Polyoxyethylen-10-stearylether; Gemisch aus Polyoxyethylen-10-stearylether und Glyceryldilaurat; Dodecyl-2-(N,N-dimethylamino)-propanoltetradecanoat und/ oder Dodecyl-2-(N,N-dimethylamino)-propianat; N-Acetylprolinatester mit > 8 C-Atomen; nichtionische Tenside, z.B. Laurylether, Ester von Polyoxyethylen; Dimethyl(arylimino)sulfuran; Gemisch aus Ölsäureanaloga und Propylenglykol; Gemisch aus Padimat O, Oktylsalicylat, Isopropylmyristat, Isopropylpalmitat, Oktylmethoxycinnimat, Laurocapram; hochdisperses Siliziumdioxid (Aerosil®); Polyoxyethylen-7-glycerol-monococoat (Cetiol® HE); 2-Octyldodecanol (Eutanol® G) oder ein Gemisch aus verschiedenen Einzelkomponenten verwenden. Es werden in dem erfindungsgemäßen transdermalen therapeutischen System hochdisperses Siliziumdioxid (Aerosil®) und/oder Polyoxyethylen-7-glycerol-monococoat (Cetiol® HE) und/ oder 2-Octyldodecanol (Eutanol® G) als fakultative(r) Permeationsförderer bevorzugt.

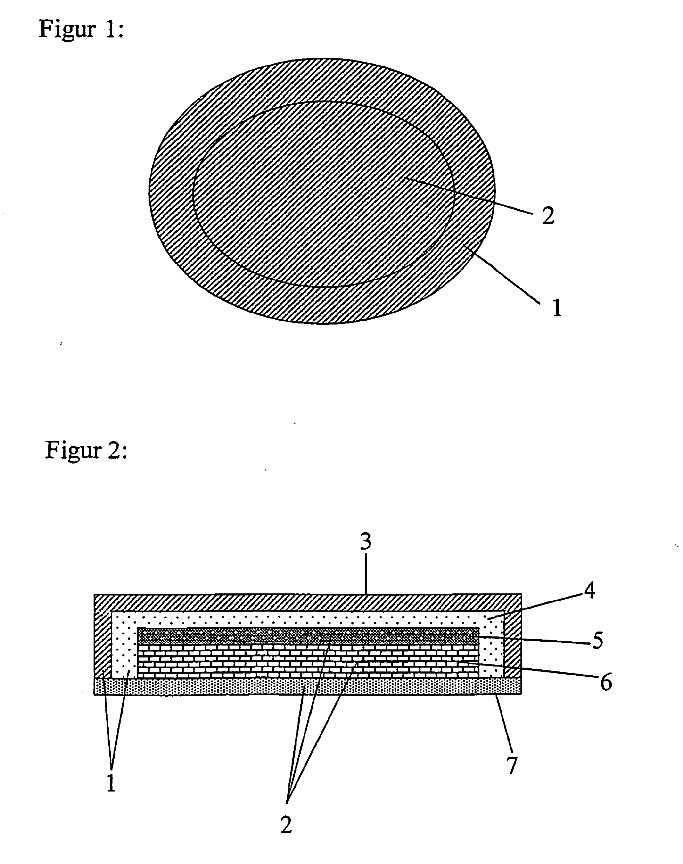

Figur 1 zeigt die Aufsicht von oben auf das transdermale therapeutische System wobei (1) das "Overtape" und (2) das übrige System darstellt.
Figur 2 zeigt das erfindungsgemäße transdermale therapeutische System mit einer selbstklebenden Matrix im Querschnitt. Die oberste Schicht stellt die für den Wirkstoff undurchlässige Deckschicht (3) dar. Darunter befindet sich eine Haftklebeschicht (4). Diese beiden Schichten bilden das "Overtape" (1). Die nächste Schicht ist abermals eine für den Wirkstoff undurchlässige Deckschicht (5). Das Material dieser Deckschicht (5) kann gleich oder verschieden zu dem Material der ersten Deckschicht (3) sein. Dann folgt die selbstklebende Matrixschicht (6), die den Wirkstoff und fakultative Permeationshemmer enthält. Das Matrixbildner ist in diesem Fall der Haftkleber. Den Abschluß bildet eine abziehbare Schutzschicht (7).

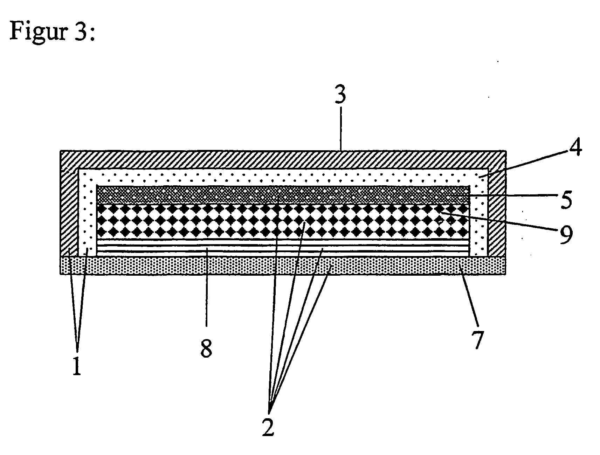

Figur 3 zeigt den Querschnitt des erfindungsgemäßen transdermalen therapeutischen Systems mit einer nicht selbstklebenden Matrixschicht (9), die mit einer separaten Haftkleberschicht (8) versehen ist.

Die Erfindung wird durch nachstehende Beispiele näher erläutert ohne aber den Erfindungsumfang damit einzuschränken.

### Beispiel 1:

**Zusammensetzung einer erfindungsgemäßen selbstklebenden Matrix für ein TTS**

| Inhaltsstoffe | Gehalt in Gew.% |
|---|---|
| Trandolaprildisäure | 10 |
| Methansulfonsäure | 2,4 |
| Aerosil® 200 | 4 |
| Cetiol® HE | 10 |
| Durotak® 387-2353 | 73,6 |

Die Gewichtsprozente beziehen sich auf das Matrixgewicht.

### Herstellungsprozeß:

Aerosil®, Cetiol® HE, Kleber (Durotak®) und Ethylacetat werden in ein geeignetes Rührgefäß eingewogen (Klebstofflösung). Parallel dazu werden in einem anderen geeigneten Rührgefäß Trandolaprildisäure und Ethylacetat eingewogen, homogenisiert, Methansulfonsäure zugegeben und so lange gerührt, bis eine klare Lösung entstanden ist (Wirkstofflösung). Dann wird die Wirkstofflösung in die Klebstofflösung überführt und homogenisiert. Diese Mischung wird auf eine Folie für die abziehbare Schutzschicht aufgetragen und im Trockenkanal getrocknet. Auf diese Matrix wird dann eine PU-Folie (z: B. Walotex 2204 ACK, 25 µm) für die wirkstoffundurchlässige Deckschicht aufgebracht. Anschließend werden die Pflaster gestanzt.

### Beispiel 2:

**Zusammensetzung einer erfindungsgemäßen selbstklebenden Matrix für ein TTS**

| Inhaltsstoffe | Gehalt in Gew.% |
|---|---|
| Ramiprildisäure | 10 |
| Methansulfonsäure | 2,5 |
| Aerosil® 200 | 4,0 |
| Cetiol® HE | 10 |
| Durotak® 387-2510 | 73,5 |

Die Gewichtsprozente beziehen sich auf das Matrixgewicht.

### Herstellungsprozeß:

Aerosil®, Cetiol® HE, Kleber (Durotak®) und Ethylacetat werden in ein geeignetes Rührgefäß eingewogen (Klebstofflösung). Parallel dazu werden in einem anderen geeigneten Rührgefäß Ramiprildisäure und Ethylacetat eingewogen, homogenisiert, Methansulfonsäure zugegeben und so lange gerührt, bis eine klare Lösung entstanden ist (Wirkstofflösung). Dann wird die Wirkstofflösung in die Klebstofflösung überführt und homogenisiert. Diese Mischung wird auf die abziehbare Schutzschicht aufgetragen und im Trockenkanal getrocknet. Die weitere Verarbeitung erfolgt wie in Beispiel 1.

### Beispiel 3:

**Zusammensetzung einer erfindungsgemäßen selbstklebenden Matrix für ein TTS**

| Inhaltsstoffe | Gehalt in Gew.% |
|---|---|
| Trandolaprilethylester | 10 |
| Aerosil® 200 | 4 |
| Cetiol® HE | 10 |
| Durotak® 387-2510 | 76 |

Die Gewichtsprozente beziehen sich auf das Matrixgewicht.

### Herstellungsprozeß:

Aerosil®, Cetiol®HE, Trandolaprilethylester und Ethylacetat werden in ein geeignetes Rührgefäß eingewogen (Wirkstofflösung). Dann wird die Wirkstofflösung in die Klebstofflösung (Durotak®) überführt und homogenisiert. Diese Mischung wird auf die abziehbare Schutzschicht aufgetragen und im Trockenkanal getrocknet. Die weitere Verarbeitung erfolgt wie in Beispiel 1.

### Beispiel 4:

**Zusammensetzung einer erfindungsgemäßen selbstklebenden Matrix für ein TTS**

| Inhaltsstoffe | Gehalt in Gew.% |
|---|---|
| Ramiprilethylester | 10 |
| Aerosil® 200 | 4 |
| Cetiol® HE | 10 |
| Durotak® 387-2510 | 76 |

Die Gewichtsprozente beziehen sich auf das Matrixgewicht.

### Herstellungsprozeß:

Aerosil®, Cetiol® HE, Ramiprilethylester und Ethylacetat werden in ein geeignetes Rührgefäß eingewogen (Wirkstofflösung). Dann wird die Wirkstofflösung in die Klebstofflösung (Durotak®) überführt und homogenisiert. Diese Mischung wird auf die abziehbare Schutzschicht aufgetragen und im Trockenkanal getrocknet. Die weitere Verarbeitung erfolgt wie in Beispiel 1.

## Patentansprüche

1. Matrixkontrolliertes transdermales therapeutisches System, umfassend (i) eine Wirkstoffundurchlässige Deckschicht, (ii) eine selbstklebende Matrixschicht oder mehrere Matrixschichten, von denen mindestens die bei Applikation des Systems freiliegende Matrixschicht selbstklebend ist, oder eine oder mehrere Matrixschichten, deren von der Deckschicht abgewandte und zur Haftung am Applikationsort vorgesehene Fläche mit einem Haftkleber beschichtet ist, wobei die Matrixschicht(en) mindestens einen ACE-Hemmer (Angiotensin Converting Enzyme-Hemmer) in Form einer Dicarbonsäure enthält, wobei der ACE-Hemmer zu einem Derivat, ausgewählt aus der folgenden Gruppe, derivatisiert ist:
Diester, und
mit säure(n) erhältliches Mono-salz,
und (iii) eine abziehbare Schutzschicht.

2. System nach Anspruch 1, g**ekennzeichnet durch** mindestens einen ACE-Hemmer aus der Gruppe Imidapril, Fosinopril, Moexipril, Perindopril, Ramipril, Spirapril, Cilazapril, Benazepril und/oder Trandolapril in Form einer Dicarbonsäure, wobei der ACE-Hemmer zu einem Diester und/oder einem mit Säure(n) gebildeten Mono-salz derivatisiert ist.

3. System nach Anspruch 1, **gekennzeichnet durch** mindestens einen ACE-Hemmer aus der Gruppe Imidapril, Fosinopril, Moexipril, Perindopril, Ramipril, Spirapril, Cilazapril und/oder Trandolapril in Form einer Dicarbonsäure, wobei der ACE-Hemmer zu einem Diester und/oder einem mit Säure(n) gebildeten Mono-salz derivatisiert ist.

4. System nach Anspruch 2 und/oder 3, **gekennzeichnet durch** Ramipril und/oder Trandolapril, insbesondere Mono-sulfonsäuresalz von Trandolaprilat und/oder Ramiprilat.

5. System nach Anspruch 2 und/oder 3, **gekennzeichnet durch** einen Ethylester des Trandolaprils und/oder Ramiprils.

6. System nach Anspruch 1, 2 und/oder 3, **dadurch gekennzeichnet, daß** der ACE-Hemmer zusätzlich zu einer ersten Estergruppe eine weitere Estergruppe aus der folgenden Gruppe trägt: Methyl-Ethyl-, Propyl-, Isopropyl-, Butyl-, tert.-Butyl-, Pentyl-, Hexyl-, Heptyl-, Octyl-, Nonan-, Decangruppe und ihre Isomeren; wobei die erste Estergruppe frei gewählt ist, wobei der ACE-Hemmer eine pharmazeutisch verträgliche Verbindung ist, oder wobei die erste und die zweite Estergruppe identisch sind.

7. System nach Anspruch 6, d**adurch gekennzeichnet, daß** die weitere Estergruppe eine Ethylgruppe ist.

8. System nach mindestens einem der Ansprüche 1 bis 7, **gekennzeichnet durch** ein Monosalz gemäß Anspruch 1, das mit einer Säure aus der folgenden Gruppe erhältlich ist: anorganische Säure, insbesondere Salzsäure, Bromwasserstoffsäure, Iodwasserstoffsäure, Salpetersäure, Schwefelsäure und Phosphorsäure, organische Carbonsäure, insbesondere Salicylsäure, Maleinsäure, Adipinsäure, Sorbinsäure, Malonsäure, 1,4-Butandisäure, Äpfelsäure, Pivalinsäure, Bernsteinsäure, Nicotinsäure, Isonicotinsäure, Furan-2-carbonsäure, Dichloressigsäure und Benzoesäure, Fettsäuren, insbesondere Laurinsäure, Myristylsäure und Ölsäure, aliphatische Sulfonsäure, insbesondere Methan-, Ethan-, Propan-, Isopropan-, Butan-, Isobutan-, Pentan-, Isopentan-, Hexan-, Heptan-, Octan-, Nonan-, Decan-, Undecan- und Dodecansulfonsäure und aromatische Sulfonsäure, insbesondere Toluol- und Benzolsulfonsäure.

9. System nach Anspruch 8, **gekennzeichnet durch** Methansulfonsäure als Säure.

10. System nach mindestens einem der vorhergehenden Ansprüche, **dadurch gekennzeichnet, daß** ACE-Hemmer
(i) vor Bildung eines Mono-salzes gemäß Anspruch 1 zur Salzbildung mit Säure(n) in equimolarem Verhältnis getrennt voneinander oder
(ii) das Mono-salz gemäß Anspruch 1 in das System eingearbeitet worden sind.

11. System nach mindestens einem der vorhergehenden Ansprüche, **gekennzeichnet durch** einen Gehalt an ACE-Hemmer (n) von 2 bis 25 Gew.-% und insbesondere 10 bis 15 Gew.-%, bezogen auf das Matrixgewicht.

12. System nach mindestens einem der vorhergehenden Ansprüche, **dadurch gekennzeichnet, daß** das System auf der Seite der Deckschicht, die der (den) Matrixschicht(en) abgewandt ist, (iv) eine Abdeckung (Overtape) aufweist,
(i) die die Deckschicht allseitig überragt und flächendeckend oder zumindest auf der die Deckschicht überragenden und in sich geschlossenen Zone mit einem Haftklebemittel versehen ist, oder.
(ii) die die Deckschicht flächendeckend abdeckt, jedoch nicht überragt, und flächendeckend mit einem Haftklebemittel versehen ist.

13. System nach Anspruch 12, **dadurch gekennzeichnet, daß** die mit einem Haftklebemittel versehene Abdeckung (Overtape) die wirkstoffundurchlässige Deckschicht vollständig abdeckt oder über der Deckschicht mit einer oder mehreren Perforationen versehen ist oder ringförmig ausgebildet ist.

14. System nach mindestens einem der Ansprüche 12 oder 13, **dadurch gekennzeichnet, daß** die wirkstoffundurchlässige Deckschicht und die mit einem Haftklebemittel versehene Abdeckung wasserdampfdurchlässig sind.

15. System nach einem der vorhergehenden Ansprüche, **dadurch gekennzeichnet, daß** die wirkstoffundurchlässige Deckschicht und die mit einem Haftklebemittel versehene Abdeckung (Overtape) aus demselben Material bestehen.

16. System nach mindestens einem der vorhergehenden Ansprüche, **dadurch gekennzeichnet, daß** die Matrixschicht(en) ein oder mehrere Permeationsförderer enthalten.

17. System nach Anspruch 16, **gekennzeichnet durch** hochdisperses Siliziumdioxid, Polyoxyethylen-7-glycerol-monococoat und/oder 2-Octyldodecanol (Eutanol G) als Permeationsförderer.

## Claims

1. Matrix-controlled transdermal therapeutic system comprising (i) an active-ingredient-impermeable cover layer, (ii) a self-adhesive matrix layer, or a plurality of matrix layers of which at least the matrix layer exposed while applying the system is self-adhesive, or one or more matrix layers whose surface remote from the cover layer and intended for adhesion at the application site is coated with an adhesive, the matrix layer(s) comprising at least one ACE inhibitor (angiotensin converting enzyme inhibitor) in the form of a dicarboxylic acid, which ACE inhibitor is derivatised to form a derivative selected from the following group:
diester, and
mono-salt obtainable with acid(s),
and (iii) a removable protective layer.

2. System according to claim 1, **characterised by** at least one ACE inhibitor from the group imidapril, fosinopril, moexipril, perindopril, ramipril, spirapril, cilazapril, benazepril and/or trandolapril in the form of a dicarboxylic acid, which ACE inhibitor is derivatised to form a diester and/or a mono-salt formed with acid(s).

3. System according to claim 1, **characterised by** at least one ACE inhibitor from the group imidapril, fosinopril, moexipril, perindopril, ramipril, spirapril, cilazapril and/or trandolapril in the form of a dicarboxylic acid, which ACE inhibitor is derivatised to form a diester and/or a mono-salt formed with acid(s).

4. System according to claim 2 and/or 3, **characterised by** ramipril and/or trandolapril, especially mono-sulphonic acid salt of trandolaprilat and/or ramiprilat.

5. System according to claim 2 and/or 3, **characterised by** an ethyl ester of trandolapril and/or ramipril.

6. System according to claim 1, 2 and/or 3, **characterised in that** the ACE inhibitor carries, in addition to a first ester group, a further ester group from the following group: methyl, ethyl, propyl, isopropyl, butyl, tert-butyl, pentyl, hexyl, heptyl, octyl, nonane, decane group and isomers thereof; the first ester group being freely selected, the ACE inhibitor being a pharmaceutically acceptable compound, or the first and second ester group being identical.

7. System according to claim 6, **characterised in that** the further ester group is an ethyl group.

8. System according to at least one of claims 1 to 7, **characterised by** a mono-salt according to claim 1 which is obtainable using an acid from the following group: inorganic acid, especially hydrochloric acid, hydrobromic acid, hydroiodic acid, nitric acid, sulphuric acid and phosphoric acid, organic carboxylic acid, especially salicylic acid, maleic acid, adipic acid, sorbic acid, malonic acid, 1,4-butanedicarboxylic acid, malic acid, pivalic acid, succinic acid, nicotinic acid, isonicotinic acid, furan-2-carboxylic acid, dichloroacetic acid and benzoic acid, fatty acids, especially lauric acid, myristic acid and oleic acid, aliphatic sulphonic acid, especially methane-, ethane-, propane-, isopropane-, butane-, isobutane-, pentane-, isopentane-, hexane-, heptane-, octane-, nonane-, decane-, undecane- and dodecane-sulphonic acid and aromatic sulphonic acid, especially toluene- and benzene-sulphonic acid.

9. System according to claim 8, **characterised by** methanesulphonic acid as acid.

10. System according to at least one of the previous claims, **characterised in that** ACE inhibitors,
(i) before formation of a mono-salt according to claim 1, together with acid(s) for salt formation, in equimolar ratio, separately from one another, or
(ii) as the mono-salt according to claim 1
have been incorporated into the system.

11. System according to at least one of the previous claims, **characterised by** a content of ACE inhibitor(s) of from 2 to 25 % by weight and especially from 10 to 15 % by weight, based on the matrix weight.

12. System according to at least one of the previous claims, **characterised in that** the system has, on that side of the cover layer which is remote from the matrix layer(s), (iv) a covering (overtape)
(i) which extends beyond the cover layer on all sides and which is provided with an adhesive that covers its surface or at least the region, in itself uninterrupted, extending beyond the cover layer, or
(ii) which covers over the surface of the cover layer but does not extend beyond it and which is provided with an adhesive that covers its surface.

13. System according to claim 12, **characterised in that** the covering (overtape) provided with an adhesive completely covers over the active-ingredient-impermeable cover layer or is provided with one or more perforations above the cover layer or is of annular shape.

14. System according to at least one of claims 12 or 13, **characterised in that** the active-ingredient-impermeable cover layer and the covering provided with an adhesive are permeable to water vapour.

15. System according to one of the previous claims, **characterised in that** the active-ingredient-impermeable cover layer and the covering (overtape) provided with an adhesive are made from the same material.

16. System according to at least one of the previous claims, **characterised in that** the matrix layer(s) comprise(s) one or more permeation enhancers.

17. System according to claim 16, **characterised by** highly disperse silicon dioxide, polyoxyethylene 7-glycerol monococoate and/or 2-octyldodecanol (Eutanol G) as permeation enhancer(s).

## Revendications

1. Système thérapeutique transdermique contrôlé par matrice, comprenant (i) une couche de recouvrement imperméable au principe actif, (ii), une couche de matrice autocollante ou plusieurs couches de matrice parmi lesquelles au moins la couche de matrice libre lors de l'application du système est autocollante, ou une ou plusieurs couches de matrice dont la surface prévue pour adhérer sur le site d'application et opposée à la couche de recouvrement est enduite d'un adhésif, la/les couche(s) de matrice contenant au moins un inhibiteur de l'ECA (inhibiteur de l'enzyme de conversion de l'angiotensine) sous la forme d'un acide dicarboxylique, l'inhibiteur de l'ECA, choisi dans le groupe suivant, étant dérivé sous forme d'un dérivat :
diester et mono-sels obtenus avec un/des acide(s)
et (iii) une couche de protection pelliculable.

2. Système selon la revendication 1, **caractérisé par** au moins un inhibiteur de l'ECA du groupe imidapril, fosinopril, moexipril, périndopril, ramipril, spirapril, cilazapril, benazépril et/ou trandolapril sous la forme d'un acide dicarboxylique, l'inhibiteur de l'ECA étant dérivé sous forme d'un diester et/ou d'un mono-sel formé avec un/des acide(s).

3. Système selon la revendication 1, **caractérisé par** au moins un inhibiteur de l'ECA du groupe imidapril, fosinopril, moexipril, périndopril, ramipril, spirapril, cilazapril et/ou trandolapril sous la forme d'un acide dicarboxylique, l'inhibiteur de l'ECA étant dérivé sous forme d'un diester et/ou d'un mono-sel formé avec un/des acide(s).

4. Système selon la revendication 2 et/ou 3, **caractérisé par** le ramipril et/ou trandolapril, en particulier le mono-sel d'acide sulfonique du trandolaprilat et/ou du ramiprilat.

5. Système selon la revendication 2 et/ou 3, **caractérisé par** un ester d'éthyle du trandolapril et/ou du ramipril.

6. Système selon la revendication 1, 2 et/ou 3, **caractérisé en ce que** l'inhibiteur de l'ECA porte en plus d'un premier groupe ester, un autre groupe ester choisi dans le groupe suivant : groupe méthyle, éthyle, propyle, isopropyle, butyle, tert-butyle, pentyle, hexyle, heptyle, octyle, nonane, décane et leurs isomères, le premier groupe ester étant choisi librement, l'inhibiteur de l'ECA étant un composé pharmaceutiquement acceptable ou le premier et le deuxième groupe ester étant identiques.

7. Système selon la revendication 6, **caractérisé en ce que** l'autre groupe ester est un groupe éthyle.

8. Système selon au moins l'une des revendications 1 à 7, **caractérisé par** un mono-sel selon la revendication 1, obtenu avec un acide choisi dans le groupe suivant : acides anorganiques, en particulier acide chlorhydrique, acide bromhydrique, acide iodhydrique, acide nitrique, acide sulfurique et acide phosphorique, acides carboxyliques organiques, en particulier acide salicylique, acide maléique, acide adipique, acide sorbique, acide malonique, diacide 1,4-butanique, acide malique, acide pivalique, acide succinique, acide nicotinique, acide isonicotinique, acide furane-2-carboxylique, acide dichloro-acétique et acide benzoïque, des acides gras, en particulier acide laurique, acide myristique et acide oléique, des acides sulfonique aliphatiques, en particulier des acides méthane-, éthane-, propane-, isopropane-, butane-, isobutane-, pentane-, isopentane-, hexane-, heptane-, octane-, nonane-, décane-, undécane- et dodécane-sulfonique et des acides sulfoniques aromatiques, en particulier les acides toluol- et benzol-sulfonique.

9. Système selon la revendication 8, **caractérisé en ce que** l'on utilise comme acide, l'acide méthane-sulfonique.

10. Système selon au moins l'une des revendications précédentes, **caractérisé en ce que** l'inhibiteur de l'ECA
(i)séparé en un rapport équimolaire avant la formation d'un mono-sel selon la revendication 1 pour la formation de sel avec un/des acide(s) ou
(ii)le mono-sel selon la revendication 1
sont intégrés dans le système.

11. Système selon au moins l'une des revendications précédentes, **caractérisé par** une teneur en inhibiteur(s) de l'ECA de 2 à 25 % en poids et en particulier de 10 à 15 % en poids par rapport au poids de la matrice.

12. Système selon au moins l'une des revendications précédentes, **caractérisé en ce que** le système présente sur le côté de la couche de revêtement qui est opposé à la/des couche(s) de matrice, (iv) un recouvrement (overtape)
(i) qui dépasse de tous les côtés de la couche de revêtement et qui est doté d'un agent adhésif, de manière couvrante, ou au moins sur la zone fermée qui dépasse de tous les côtés de la couche de revêtement,
ou
(ii) qui recouvre de manière couvrante la couche de revêtement mais ne dépasse toutefois pas et est doté d'un agent adhésif recouvrant.

13. Système selon la revendication 12, **caractérisé en ce que** le recouvrement (overtape) doté de l'agent adhésif recouvre complètement la couche de revêtement imperméable au principe actif ou est doté d'une ou de plusieurs perforations sur la couche de revêtement ou est formé de façon circulaire.

14. Système selon au moins l'une des revendications 12 ou 13, **caractérisé en ce que** la couche de revêtement imperméable au principe actif et le recouvrement doté de l'agent adhésif sont imperméables à la vapeur d'eau.

15. Système selon l'une des revendications précédentes, **caractérisé en ce que** la couche de revêtement imperméable au principe actif et le recouvrement (overtape) doté de l'agent adhésif sont constitués du même matériau.

16. Système selon au moins l'une des revendications précédentes, **caractérisé en ce que** la/les couche(s) de matrice contiennent un ou plusieurs agents stimulant la perméation.

17. Système selon la revendication 16, **caractérisé par** du dioxyde de silicium hautement dispersé, du mono-cocoate de polyoxyéthylène-7-glycérol et/ou du 2-octyldodécanol (eutanol G) comme agents stimulant la perméation.
